# EUROPEAN PATENT SPECIFICATION

(11) **EP 3 843 766 B1**
(45) Date of publication and mention of the grant of the patent: **21.10.2026**
(21) Application number: 19853394.5
(22) Date of filing: 29.08.2019
(51) Int. Cl.: A23L 2/52, A23L 2/66, A61K 36/42, A23L 33/10, A23L 33/105, A23L 33/17, A23L 33/185, A61K 31/4045, A61K 31/405, A61K 36/00, A61P 25/20, C07D 209/16, C07D 209/20, A23L 2/39

(54) **SLEEP ENHANCEMENT WITH A PROTEIN-BOUND TRYPTOPHAN AND MELATONIN MIXTURE**
SCHLAFVERBESSERUNG MIT PROTEINGEBUNDENER TRYPTOPHAN- UND MELATONINMISCHUNG
AMÉLIORATION DU SOMMEIL À L'AIDE D'UN MÉLANGE DE MÉLATONINE ET DE TRYPTOPHANE LIÉ À UNE PROTÉINE

(30) Priority: 31.08.2018 US 201862725384 P
(43) Date of publication of application: 07.07.2021
(73) Proprietor: Biosential, Inc., Toronto, ON M5X 1C7 (CA)
(72) Inventor: HUDSON, Craig, J., Toronto, ON M5X 1C7 (CA)
(74) Representative: Dennemeyer & Associates S.A.
(86) International application number: PCT/IB2019/000951
(87) International publication number: WO 2020/044115

(56) References cited:
- WO-A1-2013/074531
- WO-A1-2013/074531
- WO-A2-01/89319
- WO-A2-01/89319
- US-A1- 2014 302 170
- US-A1- 2014 302 170
- US-B2- 9 040 582
- US-B2- 9 040 582
- ANTONIO DELLA VOLPE , ANTOINETTE DE LUCIA , CLEMENTINA PIROZZI , VINCENZO PASTORE : "Comparative study between the use of melatonin and a solution with melatonin, tryptophan and vitamin B6 as an inducer of spontaneous sleep in children during an auditory response test: An alternative to commonly used sedative drugs", JOURNAL OF INTERNATIONAL ADVANCED OTOLOGY, vol. 13, no. 1, 1 April 2017 (2017-04-01), pages 69 - 73, XP055700662, DOI: 10.5152/iao.2017.3054
- NAVA ZISAPEL : "New perspective on the role of melatonin in human sleep, circadian rhythms and their regulation", BRITISH JOURNAL OF PHARMACOLOGY, vol. 175, no. 16, 20 November 2017 (2017-11-20), pages 3190 - 3199, XP055700664, DOI: 10.1111/bph.14116
- MICHAEL J SATEIA , DANIEL J BUYSSE , ANDREW D KRYSTAL , DAVID N NEUBAUER , JOHNATHAN L HEALD : "Clinical practice guideline for the pharmacologic treatment of chronic insomnia in adults : An American academy of sleep medicine clinical practice guideline", JOURNAL OF CLINICAL SLEEP MEDICINE, vol. 13, no. 2, 15 February 2017 (2017-02-15), pages 307 - 349, XP055700666, DOI: 10.5664/jcsm.6470
- SERGIO D. PAREDES, CARMEN BARRIGA , ANA B. RODRÍGUEZ: "Melatonin and tryptophan as therapeutic agents against the impairment of the sleep-wake cycle and immunosenescence due to aging in Streptopeli risoria", NEUROENDOCRINOLOGY LETTERS, vol. 28, no. 6, 1 January 2007 (2007-01-01), pages 757 - 760, XP055794550

## Description

### FIELD OF THE INVENTION

This invention describes a composition that contains natural sources of protein-bound tryptophan from plants, natural plant sources of melatonin, processes for creating said compositions, physical formulations of said compositions, and use of said compositions for enhancing sleep quality and duration in mammals.

### BACKGROUND OF THE INVENTION

Tryptophan is an essential amino acid found in many naturally-occurring plant proteins. After ingestion and absorption, tryptophan travels through the circulatory system as approximately 80% bound to serum albumin with the remaining 20% as free tryptophan. Under certain conditions tryptophan is transported into the brain and then metabolized through several biochemical pathways, one of which produces serotonin - a major neurotransmitter involved in the regulation of mood, anxiety and sleep (Pardridge, 1979). In darkness serotonin is further metabolized to melatonin - a neurohormone that regulates the sleep-wake cycle.

Melatonin is primarily synthesized in and secreted by the pineal gland - a small cone-like structure in the epithalamus of the brain (Wurtman, Axelrod & Fischer, 1964). During the night, the suprachiasmatic nucleus (SCN) of the hypothalamus stimulates the pineal gland to produce and secrete melatonin, which then acts on MT1 and MT2 receptors throughout the body to promote sleep (Dubocovich, 1988). Melatonin synthesis is regulated by feedback inhibition; at its peak concentration, melatonin suppresses SCN activity to inhibit its further synthesis, thereby regulating its own levels in the brain (Bedrosian et al., 2013). Melatonin synthesis and release therefore follow a circadian pattern, with a peak amplitude plasma concentration maintained at 25-120 pg/mL throughout the night (Waldhauser & Dietzel, 1985; Lewy & Newsome, 1983).

The hypnotic effects of both tryptophan and melatonin supplementation are well-studied. The ingestion of a sufficient quantity of tryptophan per se consistently reduces sleep latency, i.e. the time taken to fall asleep from "lights out", and improves sleep quality through enhanced sleep architecture (Boman, 1988). The hypnotic effect of tryptophan follows a dose-response curve, on which a minimum of 250 mg can improve sleep in subjects with mild insomnia or longer-than-average sleep latency (Hartmann and Spinweber, 1976; Hartmann, 1982); however, doses of 1000 mg produce more consistent improvements on sleep quality (Schneider-Helmut and Spinweber, 1986). Higher doses of tryptophan (i.e. 2,000-12,000 mg) produce little extra benefit, and doses above 12,000 mg have shown to disrupt sleep architecture (Griffiths et al., 1972). Likewise, the ingestion of a sufficient quantity of melatonin has been shown to reduce sleep latency, increase sleep duration and efficiency, and regulate the sleep-wake cycle (Dawson & Encel, 1993). The sleep-promoting effects of melatonin also exhibit a dose-response relationship, where modest increases above 1 to 5 mg produce minimal benefit. (Sack et al., 1997; Dawson & Encel, 1993). Melatonin is currently sold as an over-the-counter dietary supplement in North America.

Despite their individual hypnotic effects, the combination of tryptophan and melatonin is not expected to have a synergistic benefit on sleep due to melatonin feedback inhibition. Exogenous melatonin should theoretically suppress further endogenous melatonin synthesis to maintain an optimal plasma concentration. We have found, however, that the combined ingestion of protein-bound tryptophan and melatonin produced an unexpected superior benefit on subjective sleep quality to protein-bound tryptophan alone. The addition of 1 mg melatonin per 10,000 mg composition described in aforesaid WO 01/89319 has engendered improved sleep benefits compared to the previous melatonin-free mixture. Based on self-report data from 14 participants, 100% experienced reduced sleep latency and/or increased sleep duration, and 29% of participants reported more regular sleep-wake cycles. Yet further, there are no reports to date on the synergistic effect of tryptophan-bound protein with melatonin supplementation on sleep quality enhancement.

Compositions for inducing sleep or relaxation are disclosed in e.g., US 2014/302170 A1 and WO 2013/074531 A1.

### SUMMARY OF THE INVENTION

We have surprisingly discovered that the addition of plant-derived melatonin to the protein-bound tryptophan composition defined in WO 01/89319 produces enhanced sleep quality and duration in a mammal.

We have surprisingly discovered that human subjects who ingested a high protein food composition rich in both protein-bound tryptophan and plant-derived melatonin reported an increased improvement in sleep quality and duration, and decreased sleep latency, compared to protein-bound tryptophan alone. These results were obtained from self-report surveys for subjects comparing the sleep benefits before and after the addition of 1 mg of melatonin per 10,000 mg of the composition defined in WO 01/89319.

Thus, it is an object of this invention to provide plant sources of tryptophan and melatonin for combined use to enhance sleep quality and duration in mammals.

The invention provides a a composition comprising a meal based on a combination of a plant source containing protein-bound tryptophan having at least partially defatted butternut squash seed meal in an amount of 25 mg to 1000 mg of tryptophan, from 25 mg to 200 mg of glucose, a herb containing melatonin contained in 0.01% of the weight of the composition, a vitamin selected from the group consisting of vitamin B3, B6 and combinations thereof in an amount sufficient to enhance uptake of the tryptophan across the blood-brain barrier and a physiologically-acceptable diluent or carrier therefor for enhancing sleep in mammals.

The addition of plant-derived melatonin to the naturally-derived tryptophan-rich composition described in aforesaid WO 01/89319 has been found to enhance sleep quality and duration in mammals. Compositions of use in the invention comprise a plant source naturally containing protein-bound tryptophan - namely defatted butternut squash seed meal. Compositions of use in the invention also comprise a plant source naturally containing melatonin - preferably pigmented rice grains. The composition also contains a carbohydrate source, namely glucose, in an amount sufficient to facilitate the transport of tryptophan across the blood-brain barrier (BBB) and to circumvent the large neutral amino acid (LNAA) competition for BBB transport sites into the central nervous system (CNS). The composition can also optionally include physiologically acceptable vehicle(s), flavorings, colours and other nutrients. It also includes vitamins B3 and/or vitamin B6.

The invention further pertains to dietary supplements in the form of a drink mixture comprising the composition of the invention.

According to WO 01/89319, the use of second derivative spectroscopy has found that certain plant sources and specifically plant seeds contain relatively high levels of protein-bound tryptophan, and that these sources can provide tryptophan in vivo.

The use of analytical pressurized liquid extraction with high-performance liquid chromatography (HPLC) has found high levels - above 100 µg/kg - of melatonin in various types of pigmented rice grains, and particularly in polished, whole grain, aromatic, black, black glutinous, red and parboiled rice grains (Setyaningsih et al., 2015). Red rice grains contain high levels of melatonin and can be used herein as the plant source. Preferably, the starting rice grains contain at least 1 mg of melatonin. Melatonin concentration can be determined using known analytic methods, including, for example, HPLC and microwave-assisted extraction (MAE) (Setyaningsih, Palma & Barroso, 2012).

The plant source of melatonin can be, for example, but not limited to, pigmented rice, wheat, barley, oats, bread crumbs, grapes, cherries, strawberries, tomatoes, peppers, mushrooms, germinated white mustard seeds, germinated black mustard seeds and nuts. Preferably, the plant source is red rice grain as it is believed to contain the highest concentration of melatonin relative to other types of pigmented rice grains.

(The plant source can also be medicinal herbs including, but not limited to, Huang-qin and St. John's Wort flowers and leaves).

The composition of use in the practice of the invention includes a high-glycemic index carbohydrate. According to the present invention, the carbohydrate is glucose. It has been found that glucose facilitates the transport of tryptophan across the blood-brain barrier (BBB) for further metabolism into serotonin and melatonin in the central nervous system (CNS). The BBB consists of tight junctions between cerebral endothelial cells that regulate the diffusion of substances into the CNS. A series of transport mechanisms within the BBB selectively facilitate the movement of particular molecules into the CNS. The large neutral amino acid transporter (LAT) is one such mechanism that transports large neutral amino acids (LNAAs), including tryptophan, across the BBB. LNAAs compete for access to LAT, which prevents tryptophan transport to the brain. High-glycemic index carbohydrates can facilitate the uptake of tryptophan across the BBB relative to other LNAAs. The carbohydrate triggers the release of insulin, which shunts circulating LNAAs into skeletal muscle. Tryptophan, however, is not shunted, and remains in the bloodstream to more easily access LAT for transport across the BBB.

The composition of the invention is to be orally administered daily. The composition is formulated for the intended use of a single daily administration prior to bedtime in the absence of daylight.

It is noted that tryptophan supplementation has been shown to have potential side effects including drowsiness and minimal weight loss.

Serious side effects of short-term melatonin supplementation have not been reported in humans to date. Although uncommon, 1 mg to 5 mg of melatonin has been shown to produce drowsiness, headache, dizziness and nausea (Andersen et al., 2016). Melatonin supplementation during the day may result in sleepiness and hypothermia, which may be avoided with nighttime use (Cagnacci, Elliott & Yen, 1992). At nanomolar concentrations, melatonin has been shown to produce a vasoconstricting effect, whereas micromolar or millimolar concentrations of melatonin have been shown to produce vasodilation (Guardiola-Lemaitre, 1997); thus, the use of the composition in patients with cardiovascular disease should be at the discretion of a physician. The combination of melatonin with anti-inflammatory treatments may also increase the risk of developing gastric ulcerations (Guardiola-Lemaitre, 1997). Melatonin supplementation has also been shown to alter glucose metabolism by exerting an antihyperglycemic effect in rats; thus, individuals with Type I or Type II diabetes for which they receive chronic treatment of insulin or other means should be monitored by a physician for alterations in glucose metabolism (Guardiola-Lemaitre, 1997). The side effects of long-term melatonin supplementation requires further investigation.

### DETAILED DESCRIPTION OF PREFERRED EMBODIMENTS

### EXAMPLES

The procedures for obtaining compositions of use in the practise of the present invention are as described in aforesaid WO 01/89319.

A drink mixture of 10 grams, which is not according to the invention as defined by the claims, was made according to the general procedures described in aforesaid WO 01/89319 with the addition of plant-derived melatonin, having the composition:
5 gm of defatted squash seed
4.39 gm glucose ("glucose 43")
0.49 gm Chocolate flavor
0.07 gm guar gum
0.04 gm rice starch
0.001 gm plant source melatonin

### Patient Reports

We have found, however, that the combined ingestion of protein-bound tryptophan and melatonin produced an unexpected superior benefit on subjective sleep quality to protein-bound tryptophan alone. The addition of 1 mg melatonin to 10,000 mg of a protein-bound tryptophan composition described in aforesaid WO 01/89319 has produced surprising improvements in sleep quality and duration. Based on self-report data from 14 consumers, 100% experienced reduced sleep latency and/or increased sleep duration, and 29% reported more regular sleep-wake cycles with the melatonin-supplemented composition compared to the melatonin-free composition. Several self-reports also mentioned more intense dreams, increased feelings of restfulness and deeper sleeps.

Prior to treatment with 1 mg melatonin to 10,000 mg of a protein-bound tryptophan composition, these individuals all reported long-standing insomnia and a desire to treat their insomnia with methods other than sleep medications

### Examples:

One survey participant, a middle aged man from Bavaria, Germany reported issues with falling and staying asleep when consuming the melatonin-free protein-bound tryptophan composition once per day. The participant then consumed 10,000 mg of the composition with 0.01% added melatonin once before bed. After 60 days of this consumption, the participant reported decreased sleep latency and increased sleep duration. The participant also reported that other sleep-enhancing supplements and hypnotic medications were no longer necessary to remediate his or her insomnia-related issues.

Another participant, a middle aged woman from Germany reported a reduced quality of life due to drowsiness and long periods of wakefulness when regularly consuming the melatonin-free tryptophan composition. After 60 days of consuming 10 g of the 0.01% melatonin composition once before bed, the participant reported quick improvements in both sleep quality and ease of waking up in the morning.

Another German woman participant had struggled to fall asleep while regularly taking the melatonin-free tryptophan composition. The participant then consumed 10 g of the 0.01% melatonin composition once before bedtime for 60 days. The participant then reported reduced sleep latency, improved sleep quality, and longer and more intense sleep phases.

### References:

Bedrosian, T. A., Herring, K. L., Walton, J. C., Fonken, L. K., Weil, Z. M., & Nelson, R. J. (2013). Evidence for feedback control of pineal melatonin secretion. Neuroscience letters, 542, 123-125.
Boman, B. (1988). L-tryptophan: a rational anti-depressant and a natural hypnotic?. Australian and New Zealand journal of psychiatry, 22(1), 83-97.
Cagnacci A, Elliott JA, Yen SS. Melatonin: a major regulator of the circadian rhythm of core temperature in humans. J Clin Endocrinol Metab 1992; 75: 447-52.
Dawson, D., & Encel, N. (1993). Melatonin and sleep in humans. Journal of pineal research, 15(1), 1-12.
Dubocovich, M. L. (1988). Pharmacology and function of melatonin receptors. The FASEB Journal, 2(12), 2765-2773.
Klein, D. C., Coon, S. L., Roseboom, P. H., Weller, J. L., Bernard, M., Gastel, J. A., ... & Falcon, J. (1997). The melatonin rhythm-generating enzyme: molecular regulation of serotonin N-acetyltransferase in the pineal gland. Recent progress in hormone research, 52, 307-57.
Lewy, A. J., & Newsome, D. A. (1983). Different types of melatonin circadian secretory rhythms in some blind subjects. The Journal of Clinical Endocrinology & Metabolism, 56(6), 1103-1107.
Mclntyre, I. M., Norman, T. R., Burrows, G. D., & Armstrong, S. M. (1989). Human melatonin suppression by light is intensity dependent. Journal of pineal research, 6(2), 149-156.
Sack, R. L., Hughes, R. J., Edgar, D. M., & Lewy, A. J. (1997). Sleep-promoting effects of melatonin: at what dose, in whom, under what conditions, and by what mechanisms? Sleep, 20(10), 908-915.
Setyaningsih, W., Palma, M., & Barroso, C. G. (2012). A new microwave-assisted extraction method for melatonin determination in rice grains. Journal of cereal science, 56(2), 340-346.
Waldhauser, F., & Dietzel, M. (1985). Daily and annual rhythms in human melatonin secretion: role in puberty control. Annals of the New York Academy of Sciences, 453(1), 205-214.
Wurtman, R. J., Axelrod, J., & Fischer, J. E. (1964). Melatonin synthesis in the pineal gland: effect of light mediated by the sympathetic nervous system. Science, 143(3612), 1328-1329.

## Claims

1. A composition comprising a meal based on a combination of a plant source containing protein-bound tryptophan having at least partially defatted butternut squash seed meal in an amount of 25 mg to 1000 mg of tryptophan, from 25 mg to 200 mg of glucose, a herb containing melatonin contained in 0.01% of the weight of the composition, a vitamin selected from the group consisting of vitamin B3, B6 and combinations thereof in an amount sufficient to enhance uptake of the tryptophan across the blood-brain barrier and a physiologically-acceptable diluent or carrier therefor for enhancing sleep in mammals.

2. The composition as defined in Claim 1, wherein said partially defatted squash seed meal has a higher tryptophan source than said plant source.

3. The composition as defined in any one of Claims 1 to 2 in the form of a tablet, powder, suspension, liquid, capsule or gel.

4. The composition as defined in any one of Claims 1 to 3 in the form of a dietary supplement.

5. The dietary supplement as defined in Claim 4, wherein the supplement is formulated in a plurality of oral dosages for ingestion on a daily basis.

6. The dietary supplement as defined in Claim 5 wherein the supplement is provided as a powder drink mixture.

## Patentansprüche

1. Zusammensetzung, umfassend ein Mehl, das auf einer Kombination aus einer Pflanzenquelle basiert, die proteingebundenes Tryptophan enthält, und das mindestens teilweise entfettetes Butternusskürbiskernmehl in einer Menge von 25 mg bis 1000 mg Tryptophan aufweist, 25 mg bis 200 mg Glukose, ein melatoninhaltiges Kraut, das in 0,01 % des Gewichts der Zusammensetzung enthalten ist, ein Vitamin, das ausgewählt ist aus der Gruppe bestehend aus Vitamin B3, B6 und Kombinationen davon, in einer ausreichenden Menge, um die Aufnahme des Tryptophans über die Gehirn-Blut-Schranke zu verbessern, und einen phyisiologisch akzeptablen Verdünner oder Träger dafür zum Verbessern des Schlafs bei Säugetieren.

2. Zusammensetzung wie in Anspruch 1 definiert, wobei das teilweise entfettete Kürbiskernmehl eine höhere Tryptophanquelle aufweist als die Pflanzenquelle.

3. Zusammensetzung wie in einem der Ansprüche 1 bis 2 definiert, in der Form einer Tablette, eines Pulvers, einer Suspension, einer Flüssigkeit, einer Kapsel oder eines Gels.

4. Zusammensetzung wie in einem der Ansprüche 1 bis 3 definiert, in der Form eines Nahrungsergänzungsmittels.

5. Nahrungsergänzungsmittel wie in Anspruch 4 definiert, wobei das Ergänzungsmittel in einer Vielzahl von oralen Dosierungen zur Einnahme auf täglicher Basis formuliert ist.

6. Nahrungsergänzungsmittel wie in Anspruch 5 definiert, wobei das Ergänzungsmittel als eine Pulvergetränkemischung bereitgestellt ist.

## Revendications

1. Composition comprenant une farine basée sur une combinaison d'une source végétale contenant du tryptophane lié aux protéines présentant une farine de graines de courge musquée au moins partiellement dégraissée en une quantité de 25 mg à 1000 mg de tryptophane, de 25 mg à 200 mg de glucose, une plante contenant de la mélatonine contenue à raison de 0,01 % du poids de la composition, une vitamine choisie dans le groupe consistant en vitamine B3, B6 et des combinaisons de ceux-ci en une quantité suffisante pour améliorer le captage du tryptophane à travers la barrière hémato-encéphalique et un diluant ou excipient physiologiquement acceptable à cet effet pour améliorer le sommeil chez les mammifères.

2. Composition selon la revendication 1, dans laquelle ladite farine de graines de courge musquée partiellement dégraissée présente une source de tryptophane plus élevée que ladite source végétale.

3. Composition selon l'une quelconque des revendications 1 à 2 sous la forme d'un comprimé, d'une poudre, d'une suspension, d'un liquide, d'une capsule ou d'un gel.

4. Composition selon l'une quelconque des revendications 1 à 3 sous la forme d'un complément alimentaire.

5. Complément alimentaire selon la revendication 4, dans lequel le complément est formulé en une pluralité de doses orales destinées à une ingestion sur une base quotidienne.

6. Complément alimentaire selon la revendication 5, dans lequel le complément est fourni sous forme d'un mélange pour boisson en poudre.
